Europäisches Patentamt

(19) European Patent Office

Office européen des brevets

(11) Numéro de publication: **0 194 181**
**B1**

(12) # FASCICULE DE BREVET EUROPÉEN

(45) Date de publication du fascicule du brevet:
21.03.90

(21) Numéro de dépôt: 86400311.6

(22) Date de dépôt: 13.02.86

(51) Int. Cl. ⁵: **A 61 K 7/155, C 09 F 1/04**

(54) **Composition épilatoire à base de dérivés de colophane et son procédé de préparation.**

(30) Priorité: 13.02.85 FR 8502031

(43) Date de publication de la demande:
10.09.86 Bulletin 86/37

(45) Mention de la délivrance du brevet:
21.03.90 Bulletin 90/12

(84) Etats contractants désignés:
BE CH DE FR GB IT LI NL

(56) Documents cité:
BE-A-481 220
FR-A-2 546 405

(73) Titulaire: **des Garets, Christian**
**52, avenue de Saxe**
**F-75015 Paris (FR)**

(72) Inventeur: **des Garets, Christian**
**52, avenue de Saxe**
**F-75015 Paris (FR)**

(74) Mandataire: **Armengaud Ainé, Alain**
**Cabinet ARMENGAUD AINE 3 Avenue Bugeaud**
**F-75116 Paris (FR)**

LIBERGRAF, STOCKHOLM 1990

**Description**

La présente invention a pour objet une nouvelle composition épilatoire à base de dérivés de colophane ainsi que son procédé de préparation.

Les compositions épilatoires sont également désignées sous l'expression "cires à épiler" et celle-ci sera utilisée ci-après pour les désigner.

Les cires à épiler doivent être d'une application facile, à une température la plus basse possible, et permettre une bonne adhésion aux poils ainsi qu'à la peau, ceci de façon à permettre l'arrachage de la cire à l'aide de feuilles par exemple à l'aide de bandes de papier cellophane.

Les cires à épiler doivent par ailleurs ne pas provoquer d'irritation lors de l'application et à l'arrachage, et conférer à la peau une sensation de douceur.

Diverses compositions épilatoires ont jusqu'ici été proposées, toutes étant à base de colophane ou de dérivés de colophane. On peut à ce sujet citer les cires à épiler constituées d'un mélange de colophane et de cire d'abeille, la colophane représentant environ 75 % en poids ou constituées d'un mélange de colophane et de miel et/ou de glucose.

Les cires du premier type adhèrent fortement aux poils, mais peu à la peau, ce qui ne favorise pas un bon arrachage à l'aide de bandes. A l'inverse, les cires du second type, si elles adhèrent bien à la peau et aux bandes d'arrachage, adhèrent par contre moins bien aux poils.

Dans le brevet français FR-A-2 546 405, il a été proposé une composition épilatoire constituée d'un mélange de colophane pure dans une proportion d'environ 10 % en poids.

La colophane glycérinée plus connue sous la dénomination de "Granolite" est un glycéryl ester de colophane qui dans la compostion épilatoire, permet d'éviter, d'une part, la cristallisation de la colophane en présence d'additifs aqueux, et d'autre part d'abaisser le point de fusion, d'où la possibilité d'utilisation de la cire à épiler à basse température.

Après de nouvelles recherches dans ce domaine, on vient maintenant de constater qu'il était possible d'obtenir une excellente composition épilatoire dite "basse température" et "jetable" c'est à dire non destinée à être réutilisée après filtration, en employant comme constituant principal un sel particulier de colophane ou résinate, se présentant sous forme d'une résine liquide à une température d'environ 40 - 50°C.

La présente invention a pour objet, à titre de produit industriel nouveau, une composition épilatoire caractérisée en ce qu'elle contient de la colophane pâle, un produit gras ou huileux, de la cire d'abeille et un sel de colophane constitué par un résinate de triéthanolamine, ce sel étant présent en une proportion supérieure à 50 % et de préférence comprise entre 60 et 80 % en poids par rapport au poids total de la composition.

Le résinate de triéthanolamine ou sel de triéthanolamine de la colophane est un produit connu résultant de la salification des acides de la colophane, ces derniers représentant environ 90 % de la colophane et étant essentiellement constitués d'environ 90 % d'isomères de l'acide abiétique et de 10 % d'acide dihydroabiétique et d'acide déhydroabiétique.

Comme résinate de triéthanolamine, on peut en particulier utiliser celui vendu par la Société LA CERESINE sous la dénomination commerciale de "RESINATE z-276".

Ce résinate présente les caractéristiques physicochimiques suivantes:

Point de goutte "UBBELOHDE" 49/51°C
Densité à 20°C 1,090
Indice d'acidité 107/118
Viscosité rotative à 70°C 7,5/8,5 poises (0,75/0,85 Pas)

L'emploi, dans la composition épilatoire selon l'invention, d'une proportion prépondérante de résinate de triéthanolamine permet d'obtenir une bonne adhérence aux poils et à la peau, favorisant l'arrachement des poils en une seule application sans les casser, et permet par ailleurs d'utiliser la composition sour forme d'une couche très fine, ce qui conduit à une épilation dans des conditions très économiques.

En plus du résinate de triéthanolamine, la composition contient en outre de préférence de la colophane pâle dans une proportion d'environ 5 à 30 % en poids, un produit gras ou huileux dans une proportion de 4 à 20 % en poids ainsi qu'une cire telle que la cire d'abeille, la cire de carnauba ou la cire de candellila, mais de préférence de la cire d'abeille blanchie au soleil dans une proportion de 1 à 8 % en poids par rapport au poids total de la composition.

De préférence, le produit gras ou huileux est constitué d'une part d'une graisse telle que la lanoline et d'autre part d'une huile synthétique, minérale ou végétale, de préférence végétale. Selon cette forme de réalisation, la graisse est présente dans la composition épilatoire dans une proportion de 0,4 à 4,5 % en poids et l'huile dans une proportion de 4 à 15 % en poids par rapport au poids total de la composition.

L'huile cosmétique peut être une huile animale ou végétale par exemple: l'huile de tournesol, l'huile d'olive, l'huile d'amande douce, l'huile d'arachide, l'huile de soja, l'huile de colza, l'huile de maïs, l'huile de palme, l'huile d'avocat, l'huile de germes de blé ou encore l'huile de coco.

Selon une forme de réalisation particulièrement préférée des compositions épilatoires selon l'invention, l'huile végétale est de l'huile de colza.

La composition épilatoire selon l'invention peut en outre contenir un agent opacifiant tel que par exemple de l'oxyde de titane dans une proportion de 0,2 à 3 % en poids.

2

Selon un mode préféré de réalisation de la composition épilatoire selon l'invention, celle-ci est essentiellement constituée de:

| | |
|---|---|
| —Résinate de triéthanolamine | 60 - 85 % |
| —Colophane pâle | 5 - 30 % |
| —Huile végétale | 4 - 15 % |
| —Lanoline | 0,4 - 4,5 % |
| —Cire d'abeille | 1 - 8 % |
| —Oxyde de titane | 0,2 - 3 % |

Les compositions épilatoires peuvent en outre contenir un ester d'alcools polyhydriques de la colophane tel que par exemple le glycéryl ester de colophane ou colophane glycérinée en une proportion inférieure à 10 % en poids mais de préférence comprise entre 0,5 et 8 % en poids.

Comme colophane glycérinée, on peut notamment mentionner celle vendue sous la dénomination de "Granolite" par la Société "ETABLISSEMENTS GRANEL (SEG)".

Il va de soi que d'autres ingrédients peuvent être incorporés dans la composition épilatoire en particulier des parfums, des agents conservateurs, des colorants ainsi que diverses substances susceptibles de conférer à la composition des propriétés adoucissantes, dermopurifiantes et anti-inflammatoires notamment des extraits aqueux d'hélichryse et de calendula ou de l'azulène.

On va maintenant donner à titre d'illustration et sans aucun caractère limitatif plusieurs exemples de composition épilatoire selon l'invention.

**Exemple 1**

| | |
|---|---|
| — Résinate de triéthanolamine (RESINATE Z 296) | 77g |
| — Colophane pâle | 11g |
| — Huile de colza | 8g |
| — Lanoline | 0,65g |
| — Cire d'abeille | 2,7g |
| — Oxyde de titane | 0,65g |

**Exemple 2**

| | |
|---|---|
| — Résinate de triéthanolamine | 65g |
| — Colophane pâle | 18g |
| — Huile de tournesol | 9g |
| — Lanoline | 1,8g |
| — Cire d'abeille | 4,5g |
| — Oxyde de titane | 1,7g |

**Exemple 3**

| | |
|---|---|
| — Résinate de triéthanolamine | 63g |
| — Colophane pâle | 25g |
| — Huile de soja (ou huile d'avocat) | 6,5g |
| — Lanoline | 1,6g |
| — Cire d'abeille | 3,8g |
| — Parfum | 0,1g |

**Exemple 4**

| | |
|---|---|
| — Résinate de triéthanolamine | 80g |
| — Colophane pâle | 8g |
| — Huile d'olive | 5g |
| — Lanoline | 1,5g |
| — Cire de carnauba | 2,5g |
| — Colophane glycérinée (Granolite) | 2,5g |
| — Oxyde de titane | 0,5g |

**Exemple 5**

| | |
|---|---|
| — Résinate de triéthanolamine | 75g |
| — Colophane pâle | 12g |

- Huile de colza . . . . . . . . . . . . . . . . . 8g
- Lanoline . . . . . . . . . . . . . . . . . . . . 0,8g
- Cire de candellila . . . . . . . . . . . . . . 3,2g
- Parfum . . . . . . . . . . . . . . . . . . . . . 0,1g
- Oxyde de titane . . . . . . . . . . . . . . . 0,9g

La présente invention a également pour objet le procédé de préparation de la composition épilatoire selon l'invention.

Ce procédé consiste à préparer tout d'abord un mélange A en chauffant pendant environ 6 heures à 120 - 130°C la colophane pâle, la cire et éventuellement la graisse telle que la lanoline et d'autre part un mélange B en faisant fondre le résinate de triéthanolamine en présence de l'huile à une température d'environ 100°C et qu'après avoir obtenu ces deux mélanges, on mixe sous agitation les mélanges A et B en maintenant la température inférieure à 100°C et que l'on ajoute éventuellement l'oxyde de titane ainsi que les autres ingrédients de la composition épilatoire.

Le mélange une fois terminé, on le coule alors dans des pots et on laisse refroidir à température ambiante.

La présente invention a également pour objet un procédé d'épilation, ce procédé consistant à préchauffer la composition épilatoire selon l'invention à une température d'environ 35 - 45°C et à l'appliquer sur la peau à épiler dans le sens de la pousse des poils à l'aide d'une spatule, sous forme de bandes verticales, à apposer une feuille textile ou plastique en particulier une bande de cellophane en la faisant bien adhérer à la cire à épiler alors qu'elle est encore chaude, à laisser refroidir à une température d'environ 25°C et à retirer la bande rapidement dans le sens opposé à celui de son application.

Ce procédé permet une épilation rapide, indolore, permettant un très bon arrachage des poils et laisse la peau douce et non collante.

On peut si on le souhaite terminer le procédé d'épilation par l'application d'une lotion adoucissante et antiseptique contenant une faible proportion d'alcool.

## Revendications

1. Composition épilatoire caractérisée en ce qu'elle contient de la colophane pâle, un produit gras ou huileux, de la cire d'abeille et un sel de colophane constitué par un résinate de triéthanolamine, ce sel étant présent en une proportion supérieure à 50 % et de préférence comprise entre 60 et 80 % en poids par rapport au poids total de la composition.

2. Composition selon la revendication 1, caractérisée par le fait que le résinate de triéthanolamine présente les caractéristiques physicochimiques suivantes:

Point de goutte "UBBELOHDE" : 49/51°C
Densité à 20°C: 1,090
Indice d'acidité: 107/118
Viscosité rotative à 70°C: 7,5/8,5 poises (0,75/0,85 Pas)

3. Composition selon l'une quelconque des revendications précédentes, caractérisée par le fait qu'elle contient de 5 à 30 % en poids de colophane pâle, de 4 à 15 % en poids de produit gras ou huileux et de 1 à 8 % en poids de cire d'abeille par rapport au poids total de la composition.

4. Composition selon l'une quelconque des revendications précédentes, caractérisée par le fait que le produit gras ou huileux est constitué de 0,4 à 4,5 % en poids d'une graisse, de préférence la lanoline et de 4 à 15 % en poids d'une huile cosmétique de préférence une huile végétale.

5. Composition selon la revendication 4, caractérisée par le fait que l'huile végétale est de l'huile de colza.

6. Composition selon l'une quelconque des revendications précédentes, caractérisée par le fait que la cire est de la cire d'abeille, de la cire de carnauba ou de la cire de candellila, de préférence de la cire d'abeille blanchie au soleil.

7. Composition selon l'une quelconque des revendications précédentes, caractérisée par le fait qu'elle contient en outre de 0,2 à 3 % en poids d'oxyde de titane.

8. Composition épilatoire selon l'une quelconque des revendications précédentes, caractérisée par le fait qu'elle contient en outre un glycéryl ester de colophane en une proportion comprise entre 0,5 et 8 % en poids.

9. Procédé de préparation de la composition épilatoire selon l'une quelconque des revendications 1 à 8, caractérisé par le fait qu'il consiste à préparer tout d'abord un mélange A en chauffant pendant environ 6 heures à 120 - 130°C la colophane pâle, la cire d'abeille et éventuellement la graisse et d'autre part un mélange B en faisant fondre le résinate de triéthanolamine en présence de l'huile à une température d'environ 100°C et qu'après avoir obtenu ces deux mélanges on mixe sous agitation les mélanges A et B en maintenant la température inférieure à 100°C et que l'on ajoute éventuellement l'oxyde de titane ainsi que les autres ingrédients de la composition épilatoire.

10. Procédé d'épilation à l'aide d'une composition selon l'une quelconque des revendications 1 à 8, caractérisé par le fait qu'il consiste à préchauffer la composition épilatoire à une température d'environ 35 - 45°C et à l'appliquer sur la peau à épiler puis à apposer une feuille textile ou plastique de préférence une bande de cellophane sur la compostion alors qu'elle est encore chaude, à laisser refroidir à une température d'environ 25°C et à retirer la bande.

## Claims

1. Depilatory composition characterized in that it contains pale rosin, a fatty or oily product, beeswax and a rosin salt consisting of a triethanolamine resinate, this salt being present in a proportion greater than 50 % and preferably between 60 and 80 % by weight in relation to the total weight of the composition.

2. Composition in accordance with Claim 1, characterized by the fact that the triethanolamine resinate has the following physicochemical characteristics:

"Ubbelohde" drop point: 49 - 51°C
Density at 20°C: 1,090
Acid number: 107 - 118
Rotary viscosity at 70°C: 7.5 - 8.5 poises (0.75 - 0.85 Pas).

3. Composition in accordance with any one of the previous claims, characterized by the fact that it contains from 5 to 30 % by weight of pale rosin, from 4 to 15 % by weight of a fatty or oily product and from 1 to 8 % by weight of beeswax in relation to the total weight of the composition.

4. Composition in accordance with any one of the previous claims, characterized by the fact that the fatty or oily product consists of from 0.4 to 4.5 % of a fat, preferably lanoline, and from 4 to 15 % by weight of a cosmetic oil, preferably a vegetable oil

5. Composition in accordance with Claim 4, characterized by the fact that the vegetable oil is colza oil.

6. Composition in accordance with any one of the previous claims, characterized by the fact that the wax is beeswax, carnauba wax or candellila wax, preferably sun-bleached beeswax.

7. Composition in accordance with any one of the previous claims, characterized by the fact that is contains in addition from 0.2 to 3 % by weight of titanium oxide.

8. Depilatory composition according to any one of the previous claims, characterized by the fact that it contains in addition a glyceryl ester of rosin in a proportion of from 0.5 to 8 % by weight.

9. Process for preparation of the depilatory composition according to any one of Claims 1 to 8, characterized by the fact that it consists of first preparing a mixture A by heating pale rosin, beeswax and possibly the fat for about 6 hours at 120 - 130°C and on the other hand a mixture B by melting triethanolamine resinate in presence of the oil at a temperature of about 100°C, and that after these two mixtures have been obtained mixtures A and B are mixed with agitation while maintaining the temperature below 100°C, and that the titanium oxide and the other ingredients of the depilatory composition are possibly added.

10. Process for depilation with the aid of a composition in accordance with any one of claims 1 to 8, characterized by the fact that it consists of preheating the depilatory composition to a temperature of about 35 - 45°C and applying it to the skin to be depilated, and then affixing a textile or plastic sheet, preferably a strip of cellophane, onto the composition while it is still warm, leaving to cool to a temperature of about 25°C, and withdrawing the strip.

## Patentansprüche

1. Haarentfernungsmittel, dadurch *gekennzeichnet*, daß es blaßes Colophonium, ein fettiges oder öliges Produkt, Bienenwachs und ein aus einem Triethanolaminresinat bestehendes Colophoniumsalz enthält, wobei dieses Salz in Anteilen von mehr als 50 % und vorzugsweise in Anteilen von 60 bis 80 Gew.-% vorhanden ist, bezogen auf das Gesamtgewicht des Mittels.

2. Mittel nach Anspruch 1, dadurch *gekennzeichnet*, daß das Triethanolaminresinat die folgenden physicochemischen Eigenschaften besitzt:

Tropf. "Ubbelohde": 49/51°C

5

Dichte bei 20°C: 1,090
Säureindex: 107/118
Rotationsviskosität bei 70°C: 7,5/8,5 poises (0,75/0,85 Pas).

3. Mittel nach einem der vorhergehenden Ansprüche, dadurch *gekennzeichnet*, daß es 5 bis 30 Gew.-% blaßes Colophonium, 4 bis 15 Gew.-% eines fettigen oder öligen Produkts und 1 bis 8 Gew.-% Bienenwachs enthält, bezogen auf das Gesamtgewicht des Mittels.

4. Mittel nach einem der vorhergehenden Ansprüche, dadurch *gekennzeichnet*, daß das fettige oder ölige Produkt aus 0,4 bis 4,5 Gew.-% eines Fettes, vorzugsweise Lanolin und aus 4 bis 15 Gew.-% eines kosmetischen Öls, vorzugsweise eines pflanzlichen Öls, besteht.

5. Mittel nach Anspruch 4, dadurch *gekennzeichnet*, daß das pflanzliche Öl Kolzaöl ist.

6. Mittel nach einem der vorhergehenden Ansprüche, dadurch *gekennzeichnet*, daß es sich bei dem Wachs um Bienenwachs, Karnauberwachs oder Kandellilawachs und vorzugsweise um sonnengebleichtes Bienenwachs handelt.

7. Mittel nach einem der vorhergehenden Ansprüche, dadurch *gekennzeichnet*, daß es außerdem 0,2 bis 3 Gew.-% Titanoxid enthält.

8. Haarentfernungsmittel nach einem der vorhergehenden Ansprüche, dadurch *gekennzeichnet*, daß es außerdem einen Colophoniumglycerylester in Anteilen von 0,5 bis 8 Gew.-% enthält.

9. Verfahren zur Herstellung des Haarentfernungsmittels nach einem der Ansprüche 1 bis 8, dadurch *gekennzeichnet*, daß man zuerst eine Mischung A durch Erhitzen des blaßen Colophoniums, des Bienenwachses und gegebenenfalls des Fettes während eines Zeitraums von etwa 6 h auf 120 bis 130°C und zudem eine Mischung B durch Schmelzen des Triethanolaminresinats in Anwesenheit von Öl auf eine Temperatur von etwa 100°C herstellt und, nachdem man diese beiden Mischungen erhalten hat, diese Mischungen A und B vermischt, wobei man die Temperatur niedriger als 100°C hält und daß man gegebenenfalls Titanoxid und die weiteren Bestandteile des Haarentfernungsmittels hinzufügt.

10. Verfahren zum Entfernen von Haaren mit Hilfe eines Mittels nach einem der Ansprüche 1 bis 8, dadurch *gekennzeichnet*, daß man das Haarentfernungsmittel auf eine Temperatur von etwa 35 bis 45°C vorerhitzt und es auf die Haut, deren Haare zu entfernen sind, aufträgt, daß man ein textiles Blatt oder ein Plastikblatt und vorzugsweise einen Zellophanstreifen auf das Mittel aufdrückt, solange es noch heiß ist, daß man auf eine Temperatur von etwa 25°C abkühlen läßt und daß man den Streifen abzieht.

6